# EUROPEAN PATENT APPLICATION

(11) **EP 2 669 697 A2**
(43) Date of publication of application: **04.12.2013**
(21) Application number: 13163723.3
(22) Date of filing: 15.04.2013
(51) Int. Cl.: G01R 33/48, G01R 33/561, G01R 33/32, G01R 33/34, G01R 33/3415, A61N 1/40, A61N 5/02

(54) **An array of radiative antenna elements enclosed in a flexible dielectric medium for combined MR imaging and RF hyperthermia**

(30) Priority: 13.04.2012 EP 12164029; 12.06.2012 EP 12171684
(71) Applicant: Max-Delbrück-Centrum für Molekulare Medizin, 13125 Berlin (DE)
(72) Inventor: Niendorf, Thoralf, 52074 Aachen (DE); Winter, Lukas, 13187 Berlin (DE); Özerdem, Celal, 13187 Berlin (DE); Rieger, Jan, 13187 Berlin (DE); Hoffmann, Werner, 10318 Berlin (DE)
(74) Representative: Lange, Sven

(57) **Abstract**

The invention relates to an array of radiative antenna elements for combined MR imaging and RF hyperthermia. The antenna elements each comprise a conductive part arranged in a flexible receptacle filled with a dielectric fluid (e.g., deuteriumoxide).

## Description

The invention describes a radiative device, comprising at least one conductive part, a flexible substrate, which encloses the conductive part and an outer skin and the use of said radiative device for MR imaging or spectroscopy. The invention also describes a physiological and/or imaging measurement unit connected to said radiative device, wherein the unit comprises means for acoustic, optical, pressure, acceleration, ultrasound and/or distance measurements.

### BACKGROUND OF THE INVENTION

In the field of magnetic resonance (MR) imaging and spectroscopy, transmit radio frequency (RF) coils are used to excite protons or other nuclei which exhibit a nuclear spin at a specific frequency, which is dependent on the magnetic field strength and type of the nuclei. Transmit coils can be single RF channel configurations but might also consist of multiple transmit channels which can be driven independently from each other using different amplitude, frequency and phase settings for each channel to generate a desired RF transmit field in an object to be imaged.

One type of transmit arrays can be made of radiative elements (i.e. dipole antenna). A radiative element consists of a conductor that is mounted on a substrate. The permittivity of the substrate influences the transmission properties of the radiative element, its losses and its dimensions.

In the field of RF hyperthermia radiative elements are used in multichannel applications to increase temperature inside an object. The increase in temperature has a synergistic effect with chemotherapy and radiotherapy in the field of cancer treatment.

A hybrid birdcage coil both for imaging and RF heating is described in the state of the art. This coil is a body coil, which is not using multi-channel transmit/receive technology. Therefore no localized RF-heating (too low frequency), no flexible setup, no physiological monitoring etc. is possible.

State of the art radiative elements for MR imaging use ceramics as a substrate. However, several disadvantages arise with the proposed materials. One disadvantage is the rigid setup. Misalignment with the complex surfaces of the human body results in lower transmission efficiencies since a layer of air between the body surface and the coil influences the electromagnetic field.

Changes in the position of the ceramic substrate result in undesired changes in the resonance frequency (matching, tuning).

Another limitation in the state of the art is that due to the rigid substrate, no cooling of the surface is possible. Also the rigid setup makes the use of physiological monitoring devices difficult (i.e. ECG or other sensors below the elements).

Also no physiological parameters can be deducted through the substrate.

In addition, the state of the art radiative elements for RF heating comprises the following disadvantages:

For example a different frequency is used for RF heating and imaging, resulting in the need of additional hardware (power amplifier, antennas or filters). Imaging artifacts may arise while acquiring MR temperature maps with an MR scanner.

The medium used as a substrate prohibits the use of local transmit/receive arrays for MR imaging.

Another disadvantage is that the additional setup limits patient space drastically which most patients find uncomfortable.

Despite the usage of a flexible substrate, the setup is rigid, leading to changes in matching and tuning properties that depend on the position of the subject.

In the known state of the art mostly distilled water is used as a substrate. Distilled water, has a proton spin excitation frequency, therefore prohibiting the use of imaging.

### SUMMARY OF THE INVENTION

Therefore it was an object of the invention to provide improved means that can be used for MR imaging.

This problem is solved by the features of the independent claims. Preferred embodiments of the present invention are provided by the dependent claims.

In a first aspect, the invention relates to a radiative device, in particular for MRI, comprising at least one conductive part, a flexible substrate, which encloses the conductive part and an outer skin.

The flexible, in particular liquid, substrate enclosing the conductive part or parts bring several benefits. For example it is now possible to adjust the substrate to the human body and/or any specific body part, as the dimensions of a flexible substrate can be changed by filling in or removing liquid. This results in an improved transmission efficiency of the radiative device.

The setup of the state of the art is rigid so no physiological parameters and/or cooling of the surface and/or changing the dimensions/distance of the antennas to adjust to the imaging object is possible.

The flexible substrate is in particular a fluid, which can also be described as medium and preferably creates constant conditions for the radiative device. The substrate has the advantage that the tuning and matching of the device is not affected.

Another advantage of the invention are the properties to support simultaneous or interleaved acquisitions of more than one frequency, for example supporting simultaneous or interleaved MRI/MRS of different nuclei such a 1 H and 23Na.

The dynamic adjustment of the flexible substrate during the scan which is now possible helps to improve the imaging and/or heating properties of the radiative device. Dynamic adjustments include for example the electrical properties of the flexible substrate, the thickness of the substrate or the chemical properties of the substrate.

Another advantage is the possibility to adjust, in particular adjust dynamically, the conductive part or parts inside the flexible substrate during or prior to the scan. For example therefore the conductive parts can be pushed in the ideal position for the scan.

It is also preferred that the flexible substrate is a liquid substrate. This embodiment is beneficial because circulation inside the liquid substrate cools the surface of the object during imaging and/or heating applications.

A mixture of D20 and/or deionized/distilled water can be used i.e. with silica gel (SiO2) or other binding materials to increase the viscosity of the medium without altering its electromagnetic properties significantly. Using mixtures of deionized water and i.e. manganese chloride (MnCl2) the electromagnetic properties (increased conductivity) and MR imaging properties (extreme T2 shortening to reduce MR signal) can be altered. These substances can be combined additionally with the binding materials mentioned herein like SiO2 or agarose.

Another advantage is that it is possible to adjust the transmission and reflection parameters of the device by means of changing the temperature and hence changing the permittivity of the flexible substrate. Therefore also the frequency of the conductive parts can be altered.

The flexible substrate preferably has a high permittivity. This is a great advantage because the high permittivity enables a small device size.

It is in particular preferred that the radiative device comprises two conductive parts.

It is also advantageous that such a setup is flexible and adapts to any complex geometry and shape of any arbitrary object. An advantage of the invention is improved imaging and/or heating parameters and a higher patient comfort.

It is preferred is that the flexible substrate comprises deuteriumoxide or a mixture with deuteriumoxide. In other preferred embodiments the mixture comprises deuteriumoxide, barium titanate and/or NaCl. Barium titanate can be used to increase the permittivity or NaCl can be used to increase conductivity.

Deuteriumoxide also called heavy water or deuterium oxide or ²H₂O or D₂O, is a form of water that contains a larger than normal amount of the hydrogen isotope deuterium, rather than the common hydrogen-1 isotope that makes up most of the hydrogen in normal water. Therefore, some or most of the hydrogen atoms in heavy water contain a neutron, causing each hydrogen atom to be about twice as heavy as a normal hydrogen atom. The increased weight of the hydrogen in the water makes it slightly denser.

In the state of the art of ultra-high field imaging dipole antennas are used together with a ceramic substrate. This substrate has a permittivity of -34 increasing the element size significantly.

The radiative device according to the invention can be coupled to the surface of the irradiated object and its function is enhanced. If the flexible substrate has higher volume the radiative device can be easily positioned further from the irradiated object without impairing their functionality.

It is further preferred that the conductive part is an antenna.

In another preferred embodiment of the invention the conductive part is placed inside a covering material. In this embodiment the flexible substrate does not enclose the conductive part directly but the covering material which encloses the conductive part. It is in particular preferred that two conductive parts are placed inside one block of covering material.

It is preferred that the covering material is made of a polymer, composite and/or ceramic material. The device according to this preferred embodiment is like a bag which comprises the flexible substrate an at least one preferred more than one units of conductive part(s) placed inside covering material.

It is also preferred that the covering material itself comprises the flexible substrate, preferred deuteriumoxide. In this embodiment an additional skin is place around flexible substrate which encloses the conductive part. This unit (conductive part(s), flexible substrate and skin) is enclosed by the flexible substrate an outer skin.

In another preferred embodiment the invention concerns a radiative device wherein at least blocks of covering material comprising at least one conductive part are arrange in the device. This kind of radiative device can also be called array of radiative elements.

Hence the invention also concerns a one, two or three dimensional array of these elements (conductive part and covering material) inside a bag, a cushion or a vest or any similar cover which fits to the human body or another object to provide patient comfort and efficient transmit properties.

It is preferred that the outer skin is made of a flexible material. This embodiment is advantageous because the device is flexible and can be adjusted to the patient. Therefore the quality of the MR pictures is improved and the patients are less uncomfortable during the MRI. It is preferred that the outer skin is as a box, a bag, a cushion or a vest or any similar receptacle, which is preferably made of a flexible material and preferably fits to the human body or another object.

The radiative device of the invention can be used to transmit and/or receive MR imaging of one or multiple MR nuclei.

It is further preferred that the outer skin comprises an outlet through which the substrate can be drained and/or filled in. This embodiment is especially preferred because the flexible substrate can be replaced without much effort. Therefore the device can be used for different purposes which is very cost-effective. Another advantage is that it is now possible to adjust the substrate to the human body and/or any specific body part, as the dimensions of a flexible substrate can be changed by filling in or removing liquid.

It is also preferred that the radiative device comprises heating and/or cooling means.

Furthermore the flexible substrate can be used for cooling or heating of the surface of the object. The flexible substrate can also be used to influence the temperature of the radiative device itself and thus to drive the change in their function.

In another preferred embodiment the invention relates to the use of said radiative device for MR imaging or spectroscopy.

It was very surprising, that the radiative device can be used for MR imaging or spectroscopy. The radiative device is preferably been used for MR imaging and RF heating. In another preferred embodiment, the radiative device is used for transmitting and/or receiving MR imaging of one or multiple MR nuclei. It is also preferred that the radiative element is used for RF heating at same and/or multiple other frequencies. Different X-nuclei resonant frequency in the given field strength can be used to avoid imaging artifacts which is a great advantage compared to the state of the art.

It is preferred that the radiative device is used for MR imaging and RF heating. It is especially preferred that the device is used for MRI and RF heating at the same time. It is an advantage that due to this embodiment a low loss for high energy throughput in RF heating applications is achieved.

An advantage of the invention is that the flexible substrate can be also used to manage the distance between RF coil elements and the surface or any other part of an object to manage RF power deposition on the surface or in this object.

A main feature of the invention relates to the flexible substrate within the receptacle comprising the conductive parts to gain the benefits of multi-channel technology both for MR imaging and RF heating. The same elements are being used both for MR imaging and RF heating. Therefore no additional equipment and its disadvantages (cost, space, safety etc.) need to be implemented.

In another preferred embodiment the invention relates to the use of the radiative device for transmitting and/or receiving MR imaging of one or multiple MR nuclei.

It is also preferred that the radiative device is used for RF heating at same or multiple other frequencies.

The radiative device of the invention can be also used to measure and monitor the electrical properties of an object, which in turn can be used to manage RF transmission into this object in real-time or offline.

In the field of hyperthermia only the RF transmission is performed with radiative elements. Therefore, a different frequency with different hardware is being used. Water is being used to cool the temperature of the surface, but as the frequency is not the MR proton resonance frequency for the MR temperature measurements, this solution is not suitable for the given purpose of a hybrid applicator which combines RF heating and imaging in the same device. Additionally, with the current state-of-the-art approach space and geometry constraints are generated through the setup prohibiting the use of local multi-channel transmit and/or receive arrays and of patient comfort and space.

The preferred hybrid applicator both for localized (transmit SENSE) MR imaging and RF heating has not been described by the state of the art. The described solutions solve the problem only partly, providing either good imaging properties or good heating properties or external physiological monitoring etc.

The invention preferably uses at least one radiative device both for imaging and for heating. The device comprises a specifically defined permittivity medium (=flexible Substrate) with one or multiple (tuned) frequencies, to provide both artifact free imaging as well as desirable heating properties, ease of use, efficiency and the methodology to measure additional physiological parameter needed for metabolic and functional magnetic resonance imaging. The invention is therefore superior in many ways compared to the state of art.

In another preferred embodiment the invention relates to a physiological and/or imaging measurement unit connected to a radiative device according to the invention, wherein the unit comprises means for acoustic, pressure, ultrasound, light, UV, near infrared absorption, acceleration, optical and/or distance measurements.

Further aspect of the presented invention is that the flexible substrate is able to conduct some information about the physiological activity due to changes in pressure, colour, density, viscosity, UV absorption, near infrared absorption and/or other physics parameter in the flexible substrate. A physiological signal can be e.g. acoustic signals, bulk motion, displacement of objects/anatomy, blood pulsation or other.

The key feature of the invention - the flexible or liquid substrate - is also beneficial for this embodiment. Due to the invention acoustic measurements with acoustic wave transmission through the flexible substrate (i.e. acoustic triggering for cardiovascular MRI, Ultrasound imaging, breathing sensor, velocity measurements) are possible. Also optical measurements of physiological parameters with electromagnetic wave transmission (i.e. pulse oximetry triggering, blood oxygenation measurements) can be performed advantageously. Another kind of measurements are distance measurements with electromagnetic and/or ultrasound. These measurements are performed to evaluate the position of the human body in relation to the antenna array.

In another preferred embodiment the invention relates to a system comprising at least one radiative device of the invention and one MR apparatus, wherein the radiative device is in contact with an object to be analyzed by the MR apparatus.

Another aspect of the present invention is that the flexible substrate can be used for other imaging applications as well. Thus for example an ultrasound probe can be placed inside or outside of the flexible substrate and ideal coupling to the surface of the object is provided. In similar manner optical techniques can use the flexible substrate volume as a transmission medium to better couple to the subject.

### Examples and figures

The figures and examples provided herein represent examples of particular embodiments of the invention and are not intended to limit the scope of the invention. The figures and examples are to be considered as providing a further description of possible and potentially preferred embodiments that enhance the technical support of one or more non-limiting embodiments.

The figures illustrate basic schemes and implementations of preferred embodiments of the invention.

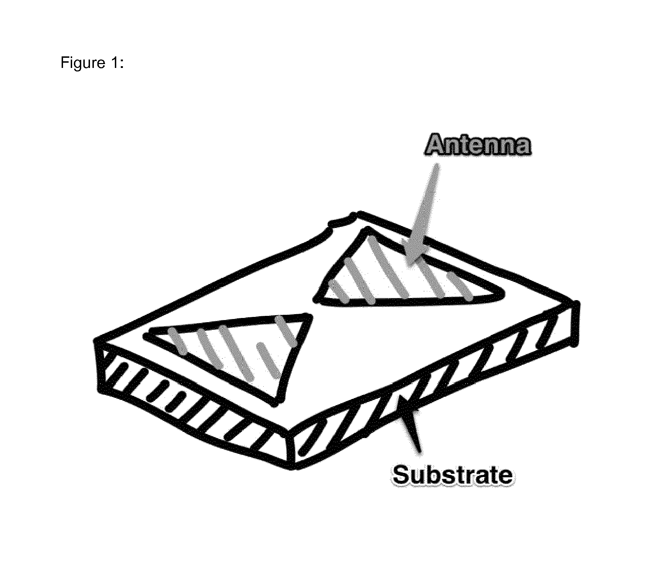

**Figure 1** shows a unit which can be part of a radiative device according to the invention. The unit consist of a conductive part (antenna) and of a substrate (polymer, composite, ceramic or any other material) and can be placed in a receptacle filled with flexible substrate.

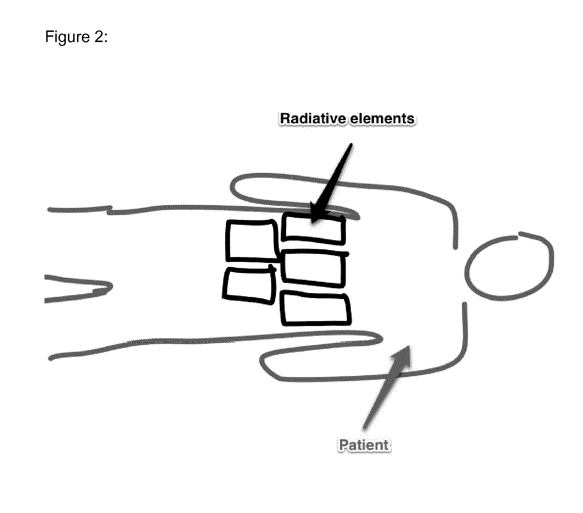

**Figure 2** shows radiative devices according to a preferred embodiment of the invention. The device can be used for imaging and heating inside of a MRI. However, further application than MRI is also preferred.

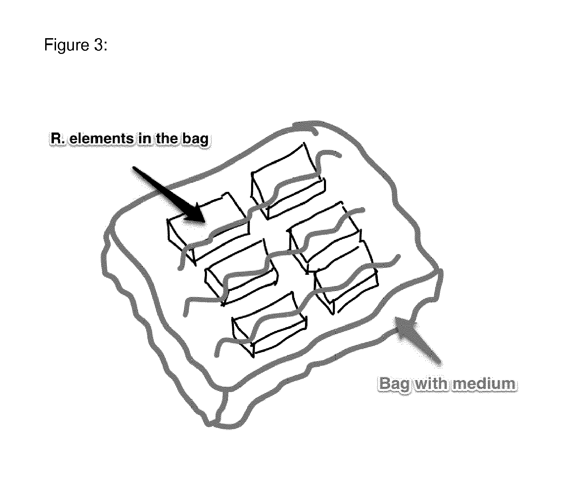

**Figure 3****:** The conductive parts or conductive parts covered with cover material can be placed inside of a receptacle (e. g. a bag) with a flexible substrate fluid (i.e. heavy water). The fluid has preferably parameters that influence the function and design of the elements - in particular their dimension.

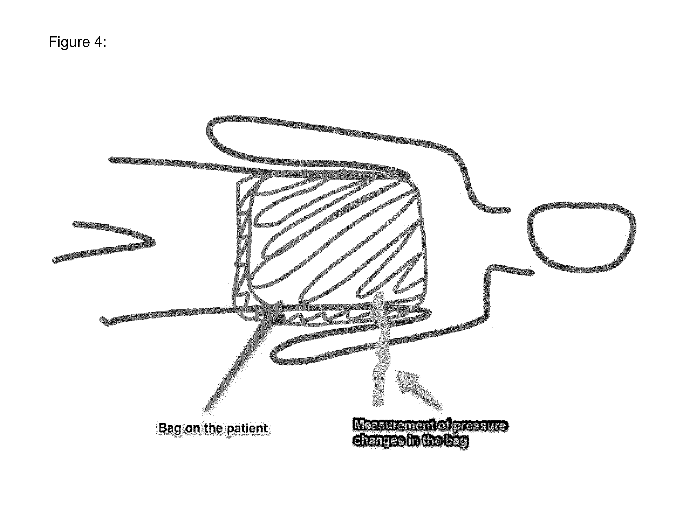

**Figure 4****:** The flexibility of the receptacle preferably improves the patient comfort. Furthermore, the fluid around the radiative elements allows for additional applications. One example is the monitoring of the heart activity. Since the receptacle lies preferably on the whole thorax it works as a big stethoscope. One could record and evaluate the heart tones as well as the overlaid breathing movement. Similar applies for the pulsation of e.g. carotids. In the case of head one could measure the pulsation of the arteries under the skin.

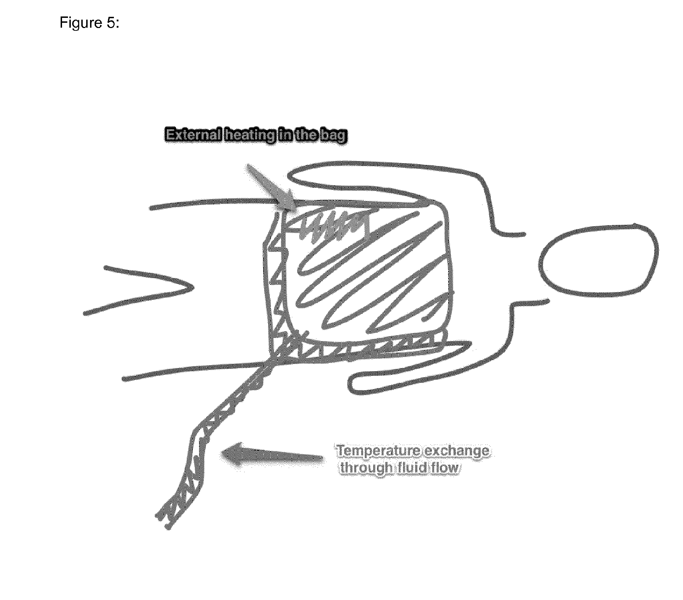

**Figure 5****:** Additionally it is preferred, that the receptacle comprises heating and/or cooling means. This would help to maintain constant temperature of the body (under normal conditions the surface might warm up) and could change the electrical parameters of the substrate and hence influence the radiation pattern of the antenna (by e. g. heating-up/chemical exchange of the bag or only of some compartments).

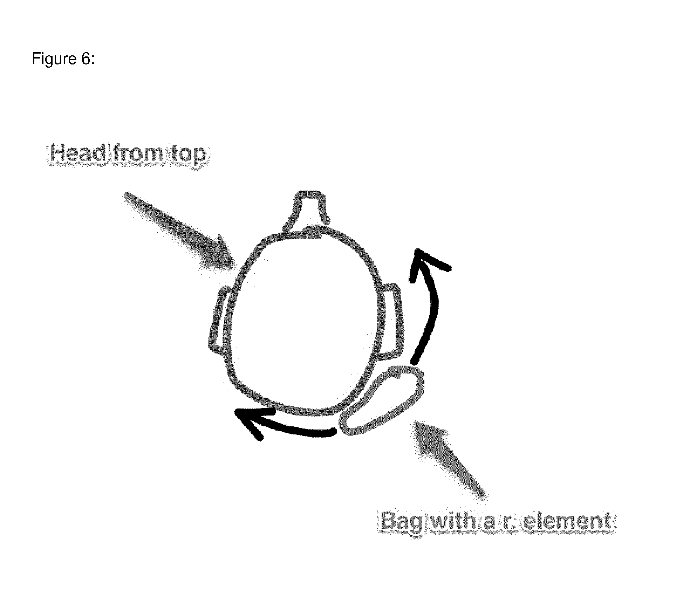

**Figure 6** shows a movable or rotating setup which is also preferred.
**Figure 7** shows results of the point SAR calculations obtained for an eight channel TX/RX configuration (Example 1).
**Figure 8** shows point SAR simulations (left) of an eight channel dipole antenna applicator at 297MHz and related temperature simulations (right) for 25W continuous wave heating for 5 minutes in an axial (a) and coronal (b) slice. Phantom properties: ε2=75, σ2=0.72 (Example 1).
**Figure 9** shows a preferred applicator design: Eight channel transmit receive applicator (a), a single element bow tie dipole antenna building block (b), the simulation setup (c) and the noise correlation matrix (d). The applicator consists preferably of eight bowtie antennas (size: 70x50mm) (Figure 9b) (Example 2).
**Figure 10** depicts imaging performance of the hybrid applicator. Simulated (a) and measured (b) B1+ maps of one element and phantom (r1). c) gradient echo image of the phantom d) Preliminary result from gradient echo imaging of a healthy brain (Example 2).
**Figure 11** shows point SAR simulations and resulting temperature simulations (25W with different phase settings in an axial (a) and coronal (b) view of the phantom (radius=90mm) (Example 2).
**Figure 12** shows a box phantom (left) filled with agarose gel (εr=75, σ=0.73 S/m) with dimensions 510x20x20mm³ (Example 3).
**Figure 13****:** A closer examination revealed good agreement between the simulated and measured B1+ maps. Figure 13 depicts the normalized simulated (left) and measured (middle) B1+ -maps. Gradient echo image (right) of the phantom (Example 3).
**Figure 14** shows: A) Simulated temperature distribution and positions of optical thermo sensors. B) Simulated (darker line) and measured (bottom line) temperature along a profile with the corresponding optical thermo sensor measurements P1 (15mm distance), P2 (40mm distance), P3 (65mm distance) and P4 (90mm distance). C) Measured temperature distribution using the PRFS method.

### Example 1:

Combining RF hyperthermia and MR imaging is conceptually appealing to pursue spatially and temporally controlled and monitored RF heating. The benefits of this approach are used as an adjunctive therapy for established cancer treatments including radiotherapy and chemotherapy [1], targeted drug delivery [2] and targeted MR contrast agent delivery [3]. The purpose of the simulation study was to evaluate the feasibility of targeted RF heating at MR frequencies ranging from 64 MHz to 500 MHz. For this purpose two different coil designs were used including a stripline array and a bowtie shaped radiative dipole antenna configuration. The simulations are an essential precursor for designing and building a hybrid applicator suitable for imaging and targeted RF heating.

### Methods:

A simulation study of an eight channel array with spin excitation frequencies at 1.5T (64MHz), 3.0T (127MHz), 7.0T (297MHz), 9.4T (400MHz) and 11.7T (500 MHz) was performed and evaluated on the ability to create a localized hotspot inside a phantom. The elements were positioned symmetrically around a cylindrical phantom with tissue properties ε1 =50.5, σ1 =0.657 S/m. Numerical electromagnetic field and transient thermal simulations were performed using CST Microwave Studio (CST GmbH, Darmstadt Germany). Post-processing of the 3D simulations was done within the RF circuit simulator of CST (Design Studio, part of CST Studio Suite 2010) as described in [4]. The mesh resolution inside the phantom was below 2x2x2mm³. To validate the simulated fields a cylindrical phantom (r1=90mm, I=250mm, ε2=75, σ2=0.72 S/m) was built. All point SAR calculations were performed for 1W accepted power at each port. Two different designs of transmit applicators were used, stripline elements and radiative dipoles. Both designs could provide a suitable setup for a hybrid applicator due to their imaging properties at ultrahigh fields [5-6]. For evaluation iso-SAR 25%, iso-SAR 50%, iso-SAR 75% and iso-SAR 90% contours were defined for the centric area inside the phantom as well as for the surface volume.

### Results:

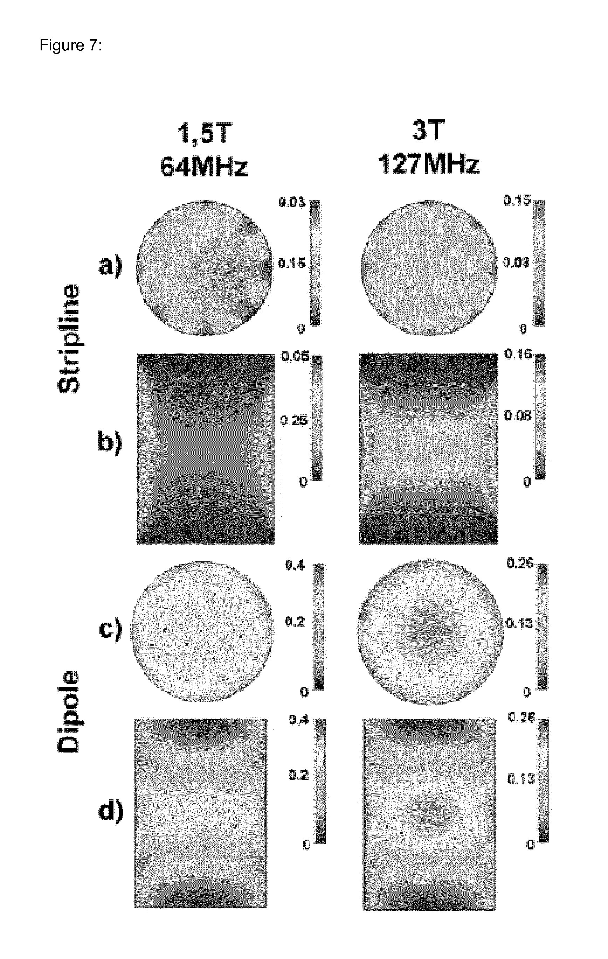

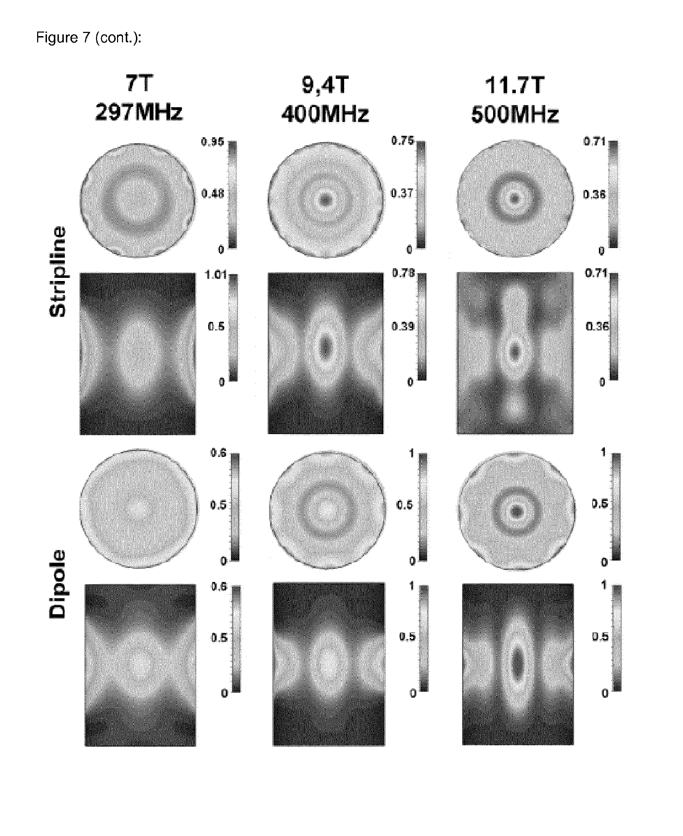

The results of the point SAR calculations obtained for an eight channel TX/RX configuration are depicted in Figure 7. At 64 MHz and 127 MHz the SAR pattern for the eight channel stripline array is distributed rather uniformly over the object. Figure 7 shows a comparison of the point SAR pattern [W/kg] of different operating frequencies for a modified stripline array in axial (a) and coronal (b) view of a cylindrical phantom (ε1=50.5, σ1=0.657 S/m) and a dipole antenna array in axial (c) and coronal (d) view.

A local SAR hot spot inside the phantom is not achievable. From 127MHz (dipole) and 297MHz (stripline) onwards focal regions of SAR increase can be formed with the eight channel array. (Figure 7, Table 1).

**Table 1 shows iso-SAR areas in [mm²] for frequencies ranging from 64 MHz to 500 MHz. iso-SAR values were derived from surface regions (surface area) and centre regions (main area) of a central axial slice of the cylindrical phantom. The missing values (-) indicate, that no iso-SAR contour could be calculated.**

| *ε₂,σ₂ | | main area | | | | surface area | |
|---|---|---|---|---|---|---|---|
| | [MHz] | 25% | 50% | 75% | 90% | 75% | 90% |
| Stripline | 64 | 172x172 | - | - | - | 20x8 | 12x4 |
| Stripline | 127 | 172x172 | - | - | - | 14x4 | 2x2 |
| Stripline | 297 | 54x56 | - | - | - | 18x4 | 2x2 |
| Stripline | 400 | 60x60 | 40x40 | 28x28 | 16x16 | 20x6 | 8x4 |
| Stripline | 500 | 48x48 | 34x34 | 20x20 | 12x12 | 14x4 | - |
| Dipole | 297 | 170x170 | 48x48 | 12x12 | - | 170x170 | 32x2 |
| Dipole* | 297 | 74x74 | 48x48 | 30x30 | 18x18 | 22x2 | - |

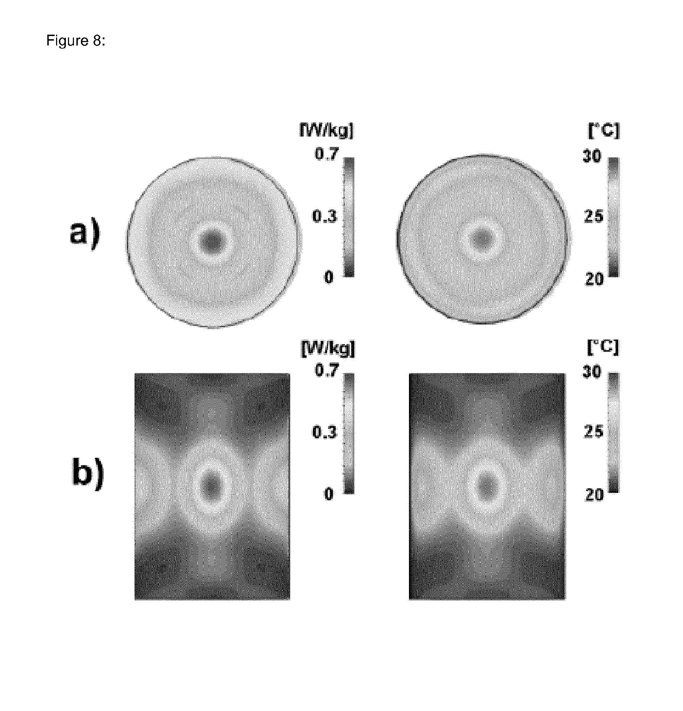

Due to the shorter wavelength inside the phantom at higher frequencies, the area of the focal hotspot is as small as 12x12mm iso-SAR 90% at 500 MHz. The dipole antenna showed improved focusing parameters at 297MHz yielding an iso-SAR 75% of 12x12mm², iso-SAR 50% of 48x48mm² in tissue and an iso-SAR 90% of 18x18mm², iso-SAR 75% of 22x22 mm² inside the phantom which comes close to that area of the hotspots achieved with the strip line configuration at 400MHz. The smallest focal SAR (iso-SAR 90%, 12x12mm²) could be achieved with the stripline array at 500MHz. The simulated SAR pattern of the dipole antenna at 297MHz generates a temperature difference inside the phantom between the central hotspot and the surface area of ΔT=5K (Figure 8). Figure 8 shows point SAR simulations (left) of an eight channel dipole antenna applicator at 297MHz and related temperature simulations (right) for 25W continuous wave heating for 5 minutes in an axial (a) and coronal (b) slice. Phantom properties: ε2=75, σ2=0.72.

The results of this example suggest that a hybrid applicator for imaging and heating is feasible. At ultrahigh fields focal hotspots can be created using a coil design which supports both RF heating as well as imaging. The size of the focal hotspot decreases with the decrease in the wavelength. Dipole antennas show improved properties for localized RF heating compared to a stripline design at 297MHz. A larger number of channels will be beneficial to further reduce surface SAR values. The same approach would increase the degree of freedom for controlling and steering the geometry and position of focal SAR hotspots and an extension towards a two-dimensional array would allow a three-dimensional steering of these focal SAR hotspots. The results can be used to build a hybrid applicator for preferably 7.0T both for imaging and RF-heating.

### References for example 1:

[1] Issels, R.D., et al., Lancet Oncol, 2010;
[2] de Smet, M., et al., JCR, 2010;
[3] Peller, M., et al., Inv Radiol, 2008;
[4] Kozlov, M. and R. Turner, JMRI, 2009;
[5] Gregor Adriany, P., et al., MRM, 2005;
[6] Raaijmakers, A., et al., MRM, 2011

### Example 2:

Recent reports demonstrate that radiative dipole antennas are beneficial for ultrahigh field (7.0T) imaging since they provide enhanced B1+ efficiency for deep lying regions and a nearly symmetrical B1+ field distribution [1]. Also, it has been shown that electric-dipole current components dominate the ultimate intrinsic signal to noise ratio (SNR) at ultrahigh fields [2]. Radiative elements are commonly used for radiofrequency (RF) hyperthermia induced targeted heating of biological tissue. This approach is used at lower fields as an adjunctive therapy for established cancer treatments including radiotherapy and chemotherapy [3-4]. Combining RF hyperthermia and MR imaging is conceptually appealing to pursue spatially and temporally controlled and monitored RF heating. The traits of ultrahigh fields can be put to use including an improved baseline SNR for high spatial resolution MRI and RF technology driven by the move towards multi-transmit/receive (TX/RX) architecture. Since the RF spin-excitation frequency (297 MHz) exceeds that of conventional RF hyperthermia systems (<140MHz) RF hyperthermia at UHF holds the promise to further sharpen the focal width of local hotspots [5-6]. To this end, this example demonstrates the feasibility of an 8 channel TX/RX hybrid applicator tailored for imaging and targeted hyperthermia at 7.0 T.

### Methods:

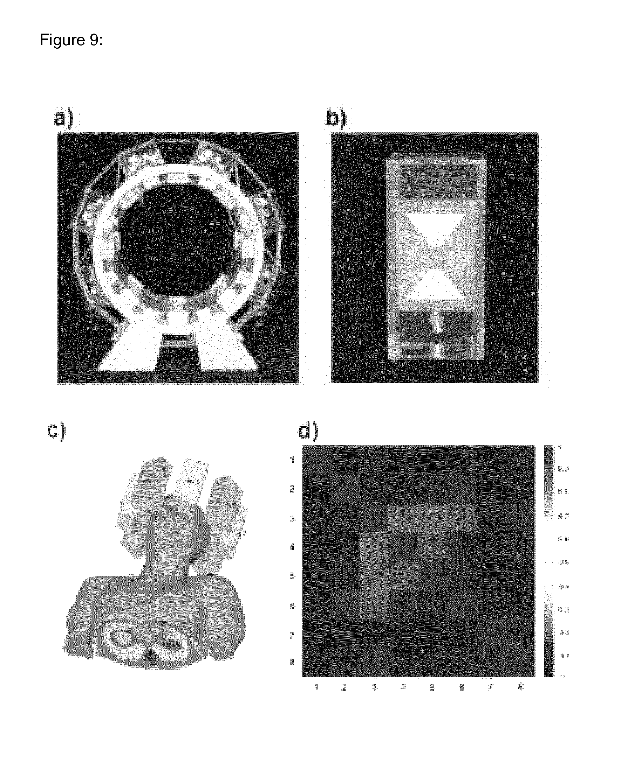

An 8 channel TX/RX applicator for imaging and RF hyperthermia at 297MHz was designed and built (Figure 9a). Figure 9 shows a preferred applicator design: Eight channel transmit receive applicator (a), a single element bow tie dipole antenna building block (b), the simulation setup (c) and the noise correlation matrix (d). The applicator consists preferably of eight bowtie antennas (size: 70x50mm) (Figure 9b). Highly surge proof (12kV) matching and tuning trim capacitors were used to allow high power throughput during RF heating. To shorten the wavelength the antenna was immersed in a water solution (75x155x40mm, 0.7g MnCl/I). The evaluation of the design was performed using numerical field simulations with CST Microwave Studio (CST GmbH, Darmstadt Germany). Imaging parameters were derived from numerical SAR10g simulations together with the voxelmodell Duke from the Virtual Family (ITIS Foundation, Zurich, Switzerland) according to IEC guidelines 60601-2-33 Ed.3. Two cylindrical phantoms (r1=90mm, r2=60mm, I=250mm, ε1=75, σ1=0.72 S/m, ε2=71, σ2=1.12 S/m) with polyethylen tubes insert were built for validation purposes [7]. B1 + maps were simulated and acquired using a pulse preparation method. Noise correlation between the elements was measured using a noise prescan. Transient temperature distributions were simulated and validated in phantom studies using the proton resonance frequency shift (PRFS) method together with optical thermo sensors (Lumasense Technologies, Santa Clara, USA) [8]. A 7.0 T whole body MRI system (Magnetom, Siemens, Erlangen, Germany) together with an 8x1 kW RF amplifier (Stolberg HF Technik AG, Stolberg-Vicht, Germany) with variable phase and amplitude settings was used for imaging and heating. A gradient echo technique was used for imaging (1.2x1.2x6mm³, TE/TR = 12/60ms). For RF heating a rectangular pulse (170V) with a duty cycle of 10% was applied.

### Results:

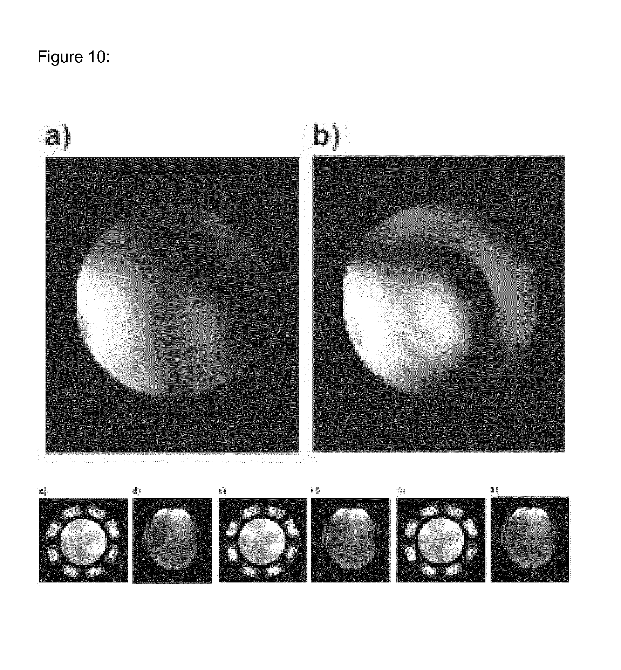

Matching and tuning parameters were below -27dB. Decoupling between elements was found to be below -21dB. Maximum measured noise correlation between elements was 0.2 (Figure 9d). Simulated and measured B1+ maps, phantom images and preliminary in vivo results are depicted in Figure 10. Figure 10 depicts imaging performance of the hybrid applicator. Simulated (a) and measured (b) B1+ maps of one element and phantom (r1). c) gradient echo image of the phantom d) Preliminary result from gradient echo imaging of a healthy brain.

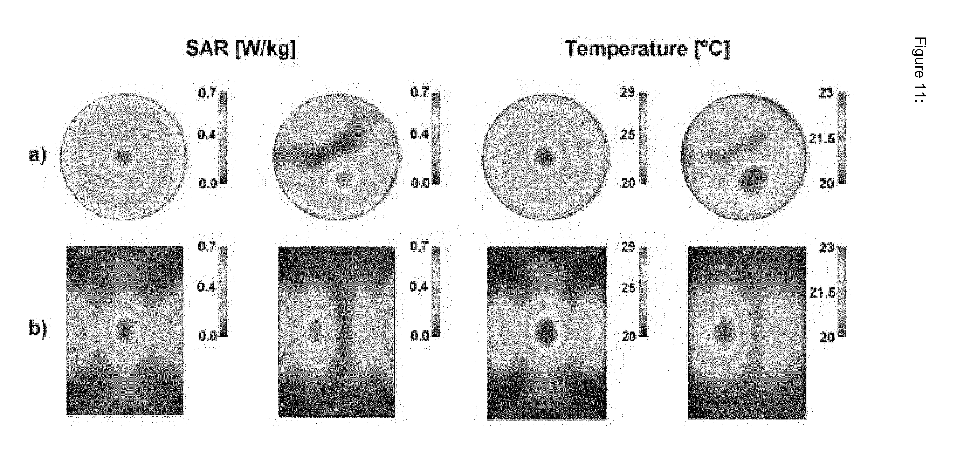

Numerical simulations showed higher SAR values in the centre of the phantom versus surface regions (Figure 11). Figure 11 shows point SAR simulations and resulting temperature simulations (25W with different phase settings in an axial (a) and coronal (b) view of the phantom (radius=90mm). The diameter of the focal width of the SAR/temperature hot spots was found to be approximately 18 mm. These SAR hotspots could be modulated and steered successfully to other arbitrary positions and regions within the phantom as demonstrated in Figure 11. For this purpose the amplitude and phase contributions of each individual bow tie antenna element were changed. The ISO SAR 90% hotspot has the dimensions of 18x18x26mm³. The temperature simulations show an increase in temperature of 9K in the centre (5min. heating, 25W CW) compared to 6K at the surface. In the experimental setup, thermo optical probes showed a temperature increase of 5K in the centre of the phantom.

The hybrid applicator showed results for imaging and RF heating. Targeted, focal deep seated SAR/temperature hotspots could be successfully generated. An increase in the number of elements could further enhance the RF modulation and steering abilities for targeted 3D RF heating. It is preferred to use heavy water to reduce power losses in the antenna and to prevent proton excitations at the antennas tip. To further cool the surface area, a water bolus together with a cooling system can be integrated into the current approach. These improvements are preferred to further drive and explore the capabilities of the proposed hybrid applicator and its implications for dedicated MR applications including targeted cancer treatment - which goes beyond current liver and abdominal applications - plus targeted drug and MR contrast agent delivery.

### References for example 2:

[1] Raaijmakers, A., et al., MRM, 2011;
[2] Lattanzi, R. and D.K. Sodickson, ISMRM Proc,2011;
[3] Nadobny, J., et al., Med Phys, 2003;
[4] Issels, R.D., et al., Lancet Oncol, 2010
[5] Dob 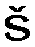 ícek Trefná, H., et al., Int J Hyperther, 2010
[6] Yang, X., et al., ISMRM Proc, 2011;
[7] Yang, Q.X., et al., MRM, 2004;
[8] Wonneberger, U., et al., JMRI, 2010

### Example 3:

Dipole antennas can be considered as an alternative for transmit-receive coil arrays tailored for ultra-high field MRI. Dipole antenna configurations offer improvements in signal-to-noise-ratio (SNR) and B1+ efficiency versus loop element or strip element configurations [1-2]. The traits of radiative elements are also useful for radio frequency (RF) hyperthermia which is of proven clinical value as an adjunctive therapy for established cancer treatments like radiotherapy and chemotherapy [3-4]. Realizing these opportunities this example describes exemplarily a bow-tie dipole antenna building block and examines its capabilities for MR imaging and its properties for RF-heating at 297MHz in an attempt to explore the feasibility of a multi-channel hybrid hyperthermia-imaging coil configuration for ultra-high fields (7.0 T). To meet these goals numerical field simulations were performed to analyse the imaging and heating properties. Validation experiments were carried out in phantoms.

### Methods:

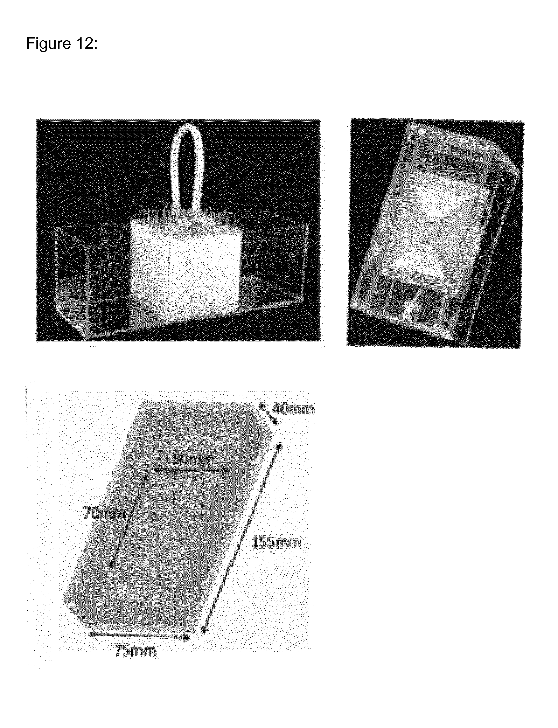

Numerical field simulations of λ/2 bow-tie shaped antenna were performed on a phantom with GST Microwave Studio (CST GmbH, Darmstadt, Germany). The simulated fields were evaluated in terms of B1+, specific absorption rate (SAR) and temperature distribution. To shorten the wavelength of the antenna at 297MHz distilled water with MnCl (0.7g/l) was used as a high permittivity medium. MnCl (0.7g/l) was added to shorten T2* and suppress the water signal for imaging. To validate the simulated data a rectangular phantom was built containing agarose gel (εᵣ=75, σ=0.73 S/m) (Figure 12). Figure 12 shows a box phantom (left) filled with agarose gel (εr=75, σ=0.73 S/m) with dimensions 510x20x20mm³. Agarose gel contains 20g/l agarose, 3.33 g/l NaCl and 0.75 g/l CuSO4. Bow tie antenna (middle) and its preferred dimensions (right). The conductivity value was chosen based in simulated data [5]. CuSO4 was added to shorten T1 for fast MR temperature measurements. A matrix of 8x8 polyethylene terephtalate (PET) tubes was integrated into the gel for temperature measurements with optical thermo sensors (LumaSense Technologies, Sata Clara, USA). For high power endurance two voltage-prrof (NMNT 10-12FS and NMNT18ENL, Voltatronics, Salisbury, U.S.A.) nonmagnetic trimmers (max. withstanding voltage 12 kV) were used as matching and tuning capacities. B1-mapping, MR imaging and RF heating were performed on a 7.0 T whole body MR system (Magnetom, Erlangen, Germany). For B1-mapping a preparation pulse method was used. A custom built RF-Amplifier (2 channels, Max. Power 2x300W (avg), frequency 100-500MHz) with a power of 100W (avg) was used for heating. MR temperature measurements were performed using the proton resonance frequency shift (PRFS) method [6]. For the heating setup the bow tie antenna and the heating phantom were placed in a birdcage coil (diameter: 27 cm, Siements, Erlangen, Germany).

### Results:

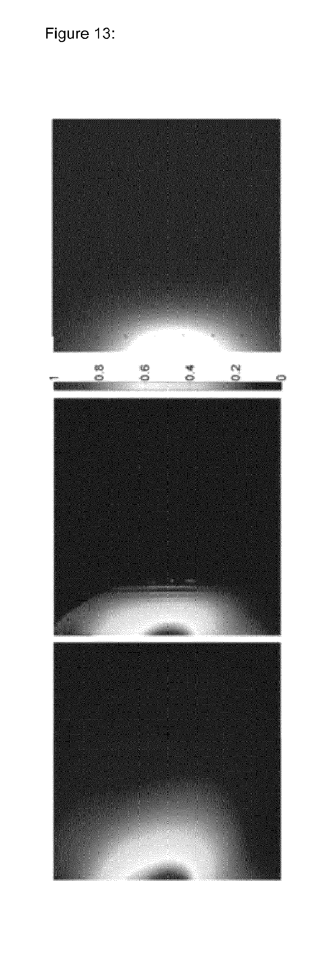

The antenna provided images of the phantom free of artefacts. The MnCl solution didn't affect the imaging properties significantly. A closer examination revealed good agreement between the simulated and measured B1+ maps (Figure 13). Figure 13 depicts the normalized simulated (left) and measured (middle) B1+ -maps. Gradient echo image (right) of the phantom.

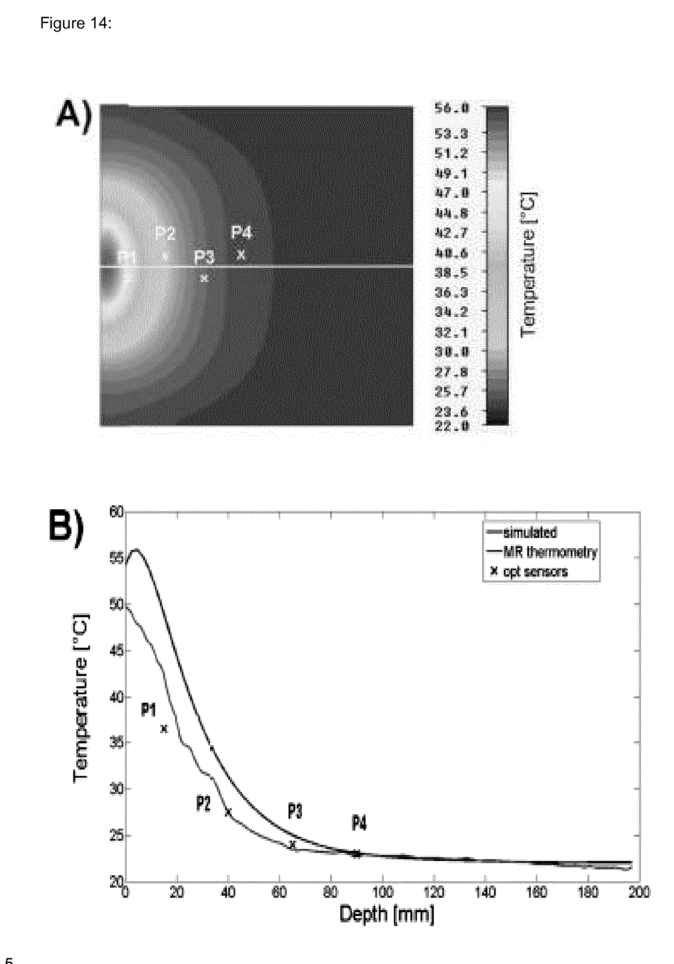

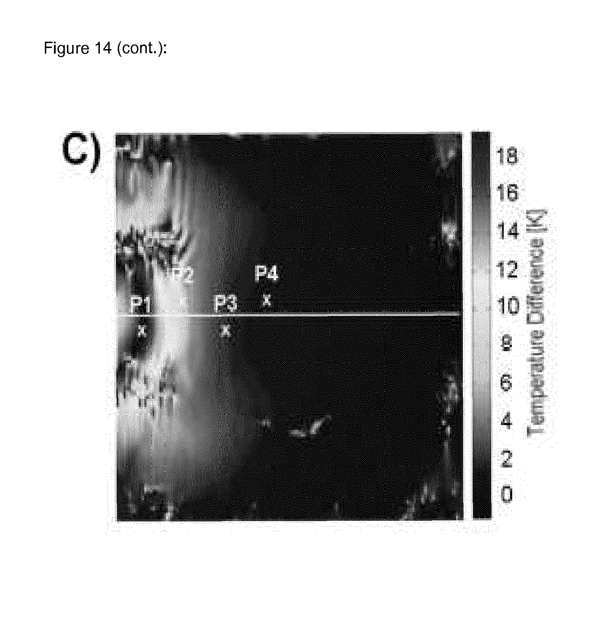

Heating of the phantom at 297 MHz was accomplished with the radiative antenna. For a heating period of 12 min using 100 Watt continuous wave a temperature increase of 5°C was observed for a region of interest placed 40 mm below the phantoms interface to the bow-tie antenna (Figure 14). Figure 14 shows: A) Simulated temperature distribution and positions of optical thermo sensors. B) Simulated (blue line) and measured (green line) temperature along a profile with the corresponding optical thermo sensor measurements P1 (15mm distance), P2 (40mm distance), P3 (65mm distance) and P4 (90mm distance). C) Measured temperature distribution using the PRFS method.

Power absorption in the permittivity medium caused a temperature increase of app. 10 degrees in this medium. For deep lying regions (distance to the surface of the antennas permittivity medium larger than 15 mm) the simulated temperature maps showed temperature changes due to RF heating similar to the measured temperature changes. For regions close to the surface of the bow-tie antennas interface a discrepancy between the simulated and the measured temperatures changes was observed.

This example proposes that bow-tie dipole antenna configurations used, afford hybrid imaging/RF heating at ultra-high fields. The TX/RX element proposed is preferred as a building block for multi-channel TX/RX hybrid imaging/RF heating architecture at 7.0 T. To further improve imaging as well as heating performance it is preferred to use deuterium oxide (heavy water) as a permittivity medium. This approach will reduce power losses in the permittivity medium and hence will help to reduce if not eliminate heating of the antenna. The MR temperature measurements need further refinement in order to produce artefact free and accurate temperature maps. Temperature simulation parameters need to be refined in order to produce accurate temperature maps in the surface area.

### References of example 3:

[1] Raaijmakers, A., et al., MRM, 2011;
[2] Lattanzi, R. and D:K. Sodickson, ISMRM Proc, 2011;
[3] Wust, P., et al., Int J of Hyperthermia, 1996;
[4] Issels, R.D., et al., Lancet Oncol, 2010;
[5] Yang, Q.X., et al., MRM, 2004;
[6] Wonneberger, U., et al., JMRI, 2010

## Claims

1. A radiative device, comprising at least one conductive part, a flexible substrate, which encloses the conductive part and an outer skin.

2. A radiative device according to claim 1, wherein the flexible substrate comprises deuteriumoxide or a mixture comprising deuteriumoxide.

3. A radiative device according to claim 2, wherein the mixture comprises barium titanate and/or NaCl.

4. A radiative device according to at least one of the preceding claims, wherein conductive part is an antenna.

5. A radiative device according to at least one of the preceding claims, wherein the conductive part is placed inside a covering material.

6. A radiative device according to the preceding claim, wherein the covering material is made of a polymer, composite and/or ceramic material.

7. A radiative device according to the preceding claim, wherein the outer skin is made of a flexible material.

8. A radiative device according to the preceding claim, wherein the outer skin comprises an outlet through which the substrate can be drained and/or filled in.

9. A radiative device according to at least one of the preceding claims, wherein the device comprises heating and/or cooling means.

10. Use of the radiative device according to at least one of the preceding claims for MR imaging or spectroscopy.

11. Use according to the preceding claim, wherein the radiative device is used for MR imaging and RF heating.

12. Use according to claim 9 or 10, wherein the radiative device is used for transmitting and/or receiving MR imaging of one or multiple MR nuclei.

13. Use according to at least one of the claims 9 to 11, wherein the radiative device is used for RF heating at same and/or multiple other frequencies.

14. A physiological and/or imaging measurement unit connected to the radiative device according to at least one of the claims 1 to 8, wherein the unit comprises means for acoustic, optical and/or distance measurements.

15. A system comprising at least one radiative device according to at least one of the claims 1 to 8 and one MR apparatus, wherein the radiative device is in contact with an object to be analyzed by the MR apparatus.
